# EUROPEAN PATENT SPECIFICATION

(11) **EP 2 112 889 B1**
(45) Date of publication and mention of the grant of the patent: **04.11.2015**
(21) Application number: 08730556.1
(22) Date of filing: 22.02.2008
(51) Int. Cl.: A23K 1/16, A23K 1/17, A23K 1/18

(54) **COMPOSITIONS AND METHODS FOR ENHANCING THE DEVELOPMENT OF GROWING ANIMALS**
ZUSAMMENSETZUNGEN UND VERFAHREN ZUR VERSTÄRKUNG DER ENTWICKLUNG WACHSENDER TIERE
COMPOSITIONS ET METHODES PERMETTANT D'AMELIORER LE DEVELOPPEMENT D'ANIMAUX EN PERIODE DE CROISSANCE

(30) Priority: 22.02.2007 US 891171 P
(43) Date of publication of application: 04.11.2009
(73) Proprietor: Hill's Pet Nutrition, Inc., Topeka, KS 66605 (US)
(72) Inventor: YAMKA, Ryan Michael, Topeka, KS 66610 (US); FRIESEN, Kim Gene, Topeka, KS 66614 (US); ZICKER, Steven Curtis, Lawrence, KS 66049 (US)
(74) Representative: Daniels, Jeffrey Nicholas
(86) International application number: PCT/US2008/054773
(87) International publication number: WO 2008/103939

(56) References cited:
- WO-A-2005/006877
- WO-A-2005/032271
- WO-A-2006/074089
- WO-A-2007/041418
- WO-A2-2006/072084
- US-A- 6 071 544
- US-A1- 2002 001 640

## Description

### FIELD OF THE INVENTION

The present invention relates to compositions and methods for enhancing the development of growing animals.

### BACKGROUND OF THE INVENTION

Commercially available canine and feline foods include compositions specially formulated to address many different nutritional needs. These include, for example, formulations designed for different breed types, sizes and body conditions. They also include formulations designed to address the nutritional needs of animals in the different stages of their life cycle. Typically, these stages include the growth, adult and senior stages of life. For example, US Patent 5,851,573 discloses a pet food composition for large breed puppies; US 6,426,100 discloses compositions to provide improved bone modeling and chondrocyte functioning in growing animals; US 6,582,752 discloses gender specific puppy food; and WO 2006/072084 discloses dietary methods to enhance the quality of life of growing animals.

Despite the availability of such pet food formulations, however, the need remains for the development of additional formulations comprising innovative ingredients and nutrients designed to enhance the development of growing animals.

Bones are metabolically active, undergoing constant resorption and redeposition of calcium. This requires the sequential and coordinated actions of osteoclasts, to remove bone (resorption), and the osteblasts to replace it (deposition). This is commonly called "bone turnover." In normal bone, following skeletal growth of an animal, the mineral deposition is in equilibrium with mineral resorption; however, in certain conditions, bone resorption exceeds bone deposition, resulting in loss of bone mineral content. High bone turnover with increased bone resorption can compromise bone strength, leading to a thinning of the bone structure. This may result in abnormal bone microarchitecture and reduced bone mineralization. Animals generally lose bone mineral content as they increase in age, resulting in osteopenia and is the major cause of osteoporosis, which may cause a greater propensity for fracture. Animals may also be pre-disposed to bone mineral content loss, as bone mineral content loss is associated with various diseases and conditions. Foods and supplements have been developed which retard bone loss, or aid in re-calcification of bone. However, there is a continuing need to develop such compositions, including compositions which promote bone formation in growing animals so as to avoid such conditions when the animal matures.

It is generally known in the art that bone desorption is accompanied by an increase in bone alkaline phosphatase ("BAP"), as calcium and phosphorous is liberated from bone. Use of various materials may effect the expression of genes involved in bone maintenance or bone formation, including bone morphogenic proteins and matrix metalloproteinases. For example, the use of carnitine for bone formation is known. However, there needs to be developed other compositions and methods which promote bone formation in animals.

Animals considered overweight and/or obese have increased in number such that it is now estimated that, in the U.S., between 25% and 40% of companion animals are considered overweight or obese. An animal is considered overweight if it weighs more than 10% above its ideal body weight, and obese if it weighs more than 15% above its ideal body weight. An animal has an ideal body weight if the animal's ribs can be felt, but not seen. Obesity in animals is implicated in increased risk of diabetes mellitus, arthritis, pancreatitis, hepatic lipidosis, orthopedic disorders, cardiovascular disease, respiratory ailments, hip dysplasia, liver disease, gastrointestinal disorders, and skin problems.

Animals such as canines and felines have been the subjects of numerous dieting schemes and exercise regimens ultimately ineffective in controlling body weight. Advances have been made in development of reduced calorie animal foods, low fat animal foods, increased nonsoluble fiber animal foods, low carbohydrate/high protein animal foods, and other foods marketed for weight control. Still, statistics indicate that as a whole, opportunities for improvement remain and further advances in the art are needed. There is, therefore, a need for new methods and compositions to improve the general fitness of animals, reducing fat, and increasing lean muscle.

Nootropic agents are known in the art, and generally include drugs and compounds which improve cognitive and neurological development. Although nootropic agents have been used for many years, some of the agents are toxic or expensive to use in food products. Thus, it is desirable to develop compositions and methods which may aid in neurological development of animals without increasing costs for manufacturing. Preferably, ingredients which are readily available in the art may be utilized to improve neurological development, however, specific formulations need to be developed before their advantages may be realized and appreciated.

### SUMMARY OF THE INVENTION

In certain aspects, the present invention relates to compositions that are useful to enhance the development of a growing animal. Particularly, the compositions of the present invention comprise one or more nutrients or bioactive substances that can enhance neurological development, bone development, and promote healthy body composition in a growing animal. In certain embodiments, the nutrients and bioactive substances include, but are not limited to, fatty acids, antioxidants, essential nutrients, amino acids, minerals and trace elements, vitamins and vitamin-like substances. Other aspects of the invention relate to methods to enhance the development of a growing animal comprising administration of effective amounts of the compositions of the present invention directly to a growing animal and/or to the dam of said animal while the animal is *in utero* and/ or is a nursling.

Thus, in one aspect, the invention relates to a Composition 1, which is a pet food composition for a therapeutic use in promoting bone development in a canine, said composition comprising:
0.8 to 1.6% methionine,
50 to 200 ppm manganese,
0.1 to 0.5% DHA,
0.1 to 0.7% EPA,
2500 to 7500 ppm choline,
wherein the canine is born of a dam fed the composition prior to and during pregnancy, and the canine is fed to composition prior to weaning.

The present invention also includes the following compositions:
1.1 Composition 1.0 comprising 1000 to 2000 ppm taurine.
1.2 Composition 1.0 or 1.1 comprising 2.5 to 6 % linoleic acid.
1.3 Any of the preceding compositions comprising 1 to 3% total n-3 fatty acids.
1.4 Any of the preceding compositions comprising 50 to 1200 IU/kg vitamin E.
1.5 Any of the preceding compositions comprising 50 to 500 ppm vitamin C.
1.6 Any of the preceding compositions comprising 2.2 to 7 g lysine/1000 kcal.
1.7 Composition 1 comprising 0 to 90% by weight of carbohydrates;
1.8 Composition 1.1 or 1.2 comprising 5% to 70% by weight protein, e.g., 20% to 50%, or 22% to 50%;
1.9 Any of the preceding compositions comprising 2% to 50% by weight of fat;
1.10 Any of the preceding compositions comprising 0.1% to 20% by weight of total dietary fiber, e.g., 1% to 11%;
1.11 The present invention also includes any of the preceding compositions comprising 0 to about 15% by weight of vitamins, minerals, and other nutrients, in varying percentages which support the nutritional needs of the animal, e.g., about 2% to about 8%;
1.12 Any of the preceding compositions comprising about 5% to about 55%, by weight of carbohydrates;
1.13 Any of the preceding compositions comprising about 20% to about 60% by weight of protein;
1.14 Any of the preceding compositions comprising about 5% to about 40%, by weight of fat, e.g., at least about 8% or about 9% to about 40% fat;
1.15 Any of the preceding compositions comprising about 200 to about 1000 IU/kg vitamin E.
1.16 Any of the preceding compositions comprising about 2.5 to about 7 g lysine/1000 kcal.
1.17 Any of the preceding compositions comprising about 100 ppm to about 500 ppm carnitine.
1.18 Any of the preceding compositions which is a dog food, e.g., a puppy food.

The present invention may also allow to enhance neurological development, enhance cognitive and motor skills, promote a healthy body composition, and/or reduce fat in a canine which is administered any one of compositions the compositions of the present invention, e.g., compositions 1.0 -1.18.

The present invention also applies when the puppy is further fed said composition post-weaning.

Other features and advantages of the present invention will be understood by reference to the detailed description of the examples that follow.

### DETAILED DESCRIPTION OF THE INVENTION

Unless defined otherwise, all technical and scientific terms used herein have the same meanings as commonly understood by one of ordinary skill in the art to which this invention belongs.

The present invention relates to any animal, preferably a mammal, more preferably a companion animal. The term "companion animal" refers to any animal that lives in close association with humans and includes, but is not limited to, canines and felines of any breed. It is contemplated herein, however, that any animal whose diet may be controlled by humans may benefit from feeding the formulations disclosed herein. These animals may include, for example, domesticated farm animals (e.g., cattle, horses, swine, etc.) as well as undomesticated animals held in captivity, e.g., in zoological parks and the like. Preferably, the animal is a canine, e.g., dog. Preferably, the canine is a growing canine, e.g., a puppy.

As used herein, "an amount effective to" or "an effective amount" to achieve a particular result, and like terms, refer to that amount of a compound, material or composition as described herein that may be effective to achieve a particularly desired biological result. As contemplated herein, such results include, for example, enhancement of neurological development, bone and joint health, immune function and/or promotion of a healthy body composition of an animal, either *in utero* and/or during its growth stage after birth, e.g., up to one year of age and beyond depending upon the species and breed of the animal, e.g., a canine. Such effective activity is achieved by administration of compositions of the present invention to the dam of said animal while the animal is *in utero* or nursing, further including the direct administration to the animal during its growth stage.

As used herein, the "enhancement" of a particular biological process or body condition in a growing animal such as described herein refers to an improvement in the biological process or body condition of a growing animal compared to a control animal. Improvement in such a process or condition may be determined by one of skill in the art. For example, an enhancement in neurological development may be indicated by better brain development, as measured by MRI spectroscopy, and/or by better cognitive and motor skill scores, compared to control animals. An enhancement in bone development may be indicated, for example, by lower bone alkaline phosphatase levels compared to control animals and/or by higher dual energy X-ray absorptiometry (DXA or DEXA) bone density scores compared to controls. The body composition of growing animals may also be assessed by DXA; bone mineral density, bone mineral content, lean mass, total fat mass, percent fat mass, lean and bone content may be assayed in an animal administered the compositions according to the methods of the present invention and compared to control animals, where animals with less percent body fat compared to controls are considered to have a healthy body composition.

As used herein, "enhancement of the development of a growing animal" or "enhanced growth" and like terms refer to an overall improvement in one or more biological processes and/or the body condition of a growing animal, including but not limited to, biological processes central to the growth and development of an organism, including, but not limited to, the biological processes described herein, e.g., bone and joint health, neurological and immune system development and body weight gain (e.g., increase in lean muscle mass instead of adipose tissue).

The "growth" life stage of an animal refers to the period from birth or weaning (approximately 8 weeks of age) to about 1 year of age or beyond, depending on the species and breed of the animal.

As used herein, the term "puppy" refers to an immature canine, typically between the ages of birth and 12 months.

As used herein, a "healthy body composition" refers to a body composition in a growing animal that comprises a normal percent body fat, e.g., a body fat of approximately 25%, with a tendency to put on weight by adding lean muscle mass rather than adipose tissue.

"Essential amino acids" as used herein refers to those amino acids that cannot be synthesized *de novo* by an organism and thus must be supplied in the diet. It is understood by one of skill in the art that the essential amino acids vary from species to species, depending upon the organism's metabolism. For example, it is generally understood that the essential amino acids for dogs and cats (and humans), are phenylalanine, leucine, methionine, lysine, isoleucine, valine, threonine, tryptophan, histidine and arginine.

As understood by one of skill in the art, a "limiting amino acid" refers to an amino acid which if present in insufficient quantities in a diet, results in the limitation in usefulness of other essential amino acids, even if the other essential amino acids are present in otherwise large enough quantities. Lysine is the limiting essential amino acid in the compositions disclosed herein. Thus, the remaining essential amino acids are quantitatively formulated or "balanced" in relationship to the amount of lysine determined critical to affect the desired biological result. As used herein, "balanced amino acids" refers to the relationship of the essential amino acid lysine to energy to assure optimal animal growth and development.

"Essential nutrients" as used herein refers to nutrients required for normal body functioning that cannot be synthesized by the body. Categories of essential nutrient include vitamin dietary minerals, fatty acid, and amino acid. It is understood by one of skill in the art that the nutrients deemed essential varies from species to species, depending upon the organism's metabolism. For example, essential nutrients for dogs and cats include Vitamins A, D, E, K, B1, B6, B12, riboflavin, niacin, pantothenic acid, folic acid, calcium, phosphorous, magnesium, sodium, potassium, chlorine, iron, copper, zinc, manganese, selenium and iodine. Choline, generally regarded as a B complex vitamin, may be included among the semi-essential nutrients. In addition, taurine, while technically not an amino acid but a derivative of cysteine, is an essential nutrient for cats.

Carnitine, also known as L-carnitine, (levocarnitine) is a quaternary ammonium compound synthesized from the amino acids lysine and methionine and is responsible for the transport of fatty acids from the cytosol into the mitochondria.

Without being limited to any theories or particular modes of action, the present invention is based on the surprising discovery that the addition of certain ingredients to pet food compositions and administration of these compositions to animals can enhance the development of a growing animal. For example, data indicate that animals fed the compositions of the present invention (or those whose dams were fed the compositions during gestation and prior to weaning but continued throughout growth of their litters), demonstrate enhanced neurological development, bone and joint health, and have overall healthier body composition.

As contemplated herein, the compositions of the present invention comprise nutritionally complete and balanced animal feed compositions. Such compositions include, among other nutrients and ingredients, recommended healthful amounts of protein, carbohydrate and fat. "Nutritionally complete and balanced animal feed compositions", as well as nutrients and ingredients suitable for animal feed compositions, and recommended amounts thereof, are familiar to one of skill in the art (see, for example, National Research Council, 2006 Nutritional Requirements for Dogs and Cats, National Academy Press, Washington D.C. or the Official Publication of the Association of American Feed Control Officials, Inc. Nutrient Requirements for Dogs and Cats 2006).

It is contemplated herein that the compositions disclosed herein may also comprise antioxidants, additives, stabilizers, thickeners, flavorants, palatability enhancers and colorants in amounts and combinations familiar to one of skill in the art. "Antioxidants" refers to a substance that is capable of reacting with or decreasing the production of free radicals and neutralizing them. Examples include, but are not limited to, beta-carotene, selenium, coenzyme Q10 (ubiquinone), lutein, tocotrienols, soy isoflavones, S-adenosylmethionine, glutathione, taurine, N-acetylcysteine, vitamin E, vitamin D, vitamin C, flavanoids, anthocyanindins, and lipoic acid.

While foods of any consistency or moisture content are contemplated, preferably the compositions of the present invention may be, for example, a wet, semi-dry or dry animal food composition. "Wet" food refers to food which is sold in cans or foil bags and has a moisture content of about 70 to about a 90%. "Dry" food refers to compositions with about 5 to about 15% moisture content and is often manufactured in the form of small bits or kibbles. Semi-dry compositions refer to compositions having about 15% to about 70% moisture. Also contemplated herein are compositions of intermediate moisture consistency and those that may comprise components of various consistency as well as components that may include more than one consistency, for example, soft, chewy meat-like particles as well as kibble having an outer cereal component and an inner cream component.

Another aspect of the invention relates to methods to enhance the development of a growing animal by administering to the animal an effective amount of a composition of the present invention. As defined above, "enhancement of the development of a growing animal" refers to an overall improvement in one or more biological processes and/or the body condition of a growing animal, including but not limited to, biological processes central to the growth and development of an organism, including, but not limited to, the biological processes described herein, e.g., bone and joint growth, neurological and immune system development and healthy body weight gain. One of skill in the art is familiar with means to assay the condition of an animal and characterize the condition as improved or not compared to a control.

The invention may also allow to enhance the neurological development of a growing animal comprising administering to said animal a composition of the present invention. For example, an enhancement in neurological development may be indicated by better cognitive and motor skill scores, compared to control animals. For instance, the state of neurological development may be assayed by measuring the level of trainability in a growing animal to which is administered a composition of the present invention and comparing levels to a suitable control animal.

In another aspect, the invention relates to a method to enhance bone and joint health in a growing animal comprising administering to said animal a composition of the present invention. An enhancement in bone and joint health may be indicated by, for example, lower bone alkaline phosphatase levels compared to control animals as it is believed that lower bone alkaline phosphatase levels are indicative of decreased bone turnover and thus a propensity of more dense bone growth. In addition, lower levels of arthritic markers, e.g., carboxy-terminal c- telopeptide of type 1 cartilage and deoxy-pyrodiniline, may indicate a decreased amounts of cartilage damage or bone damage and turnover, respectively. Bone and joint health may also be assayed using dual energy X-ray absorptiometry (DXA), with enhanced bone and joint health indicated by higher bone density scores compared to controls.

The invention may also allow to enhance immune function in a growing animal comprising administering to said animal a composition of the present invention. For example, animals with enhanced immune function may be characterized as those which demonstrate greater antibody response upon antigen challenge than controls.

In another aspect, the invention also allows to to promote a healthy body composition in a growing animal. Generally, a healthy body composition may be considered to be a body condition with normal percent body fat (e.g., approximately 25% (16-24% for BCS=3)) and/or in which the percent body fat does not exceed 27% as determined by DXA, or when an animal has a body condition score of no more than 3 as determined using, for example, the method disclosed in "Small Animal Clinical Nutrition", 4th Edition, Chapter 13 (ISBN 0-945837-05-4) or its equivalent using other conventional techniques to measure body condition score. As a rule, during any stage of an animal's life, body weight gain due to an increase in lean muscle mass is preferred to weight gain attributable to an accumulation of adipose tissue.

It is contemplated herein that the compositions of the present invention may be administered to an animal alone as a complete nutritionally balanced diet, or in conjunction with dietary supplements, vitamins and/or other nutritionally beneficial agents familiar to one of skill in the art, as part of an overall wellness program for the animal. Compositions of the invention may also be useful as a veterinary therapeutic product. As such, the compositions may optionally contain a carrier, diluent, or an excipient, the suitability of which for the intended use being familiar to one of skill in the art.

It is a feature of the invention that, in addition to administering the compositions disclosed herein directly to a growing animal, e.g., to a growing puppy or kitten, the compositions is also administered to the dam of the animal while the animal is still *in utero* and while the animal is a nursling.

The following examples further illustrate the present invention and are not intended to limit the invention. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention.

Except to the extent stated otherwise, all percentages used in this specification are weight percentages on a dry matter basis. The phrase "dry matter basis" means the component concentration in the composition after any moisture in the composition is removed.

### Example 1

Formulations to enhance the development of growing animal are disclosed herein. These compositions are developed taking into account the "ideal protein concept" (Baker and Czarnecki-Maulden, 1991 Annu. Rev. Nutr. 11:239-63).

Foods are developed for the "growth" life stage. These foods include formulations for canine growth and feline growth. The minimum nutrient recommendations for these foods, as well as the targeted values for a prototype food, are listed below in Table 1.

**Table 1. Key Nutrients for Puppy Formula**

| Nutrient | Target | Minimum | Maximum |
|---|---|---|---|
| Protein, % | 31.3 | 20 | 50 |
| Methionine, % | 1.3 | 0.8 | 1.6 |
| Manganese, ppm | 100 | 50 | 200 |
| DHA, % | 0.23 | 0.1 | 0.5 |
| EPA, % | 0.35 | 0.1 | 0.7 |
| Choline, ppm | 5190 | 2500 | 7500 |
| Taurine, ppm | 1600 | 1000 | 2000 |
| Linoleic acid, % | 4.2 | 2.5 | 6 |
| Total n-3 fatty acids, % | 1.51 | 1.0 | 3 |
| Vitamin E, IU/kg | 870 | 200 | 1200 |
| Vitamin C, ppm | 90 | 50 | 500 |
| Carnitine, ppm | 300 | 100 | 500 |
| Lysine, g/1000 kcal | 3.5 | 2.5 | 7 |

### Example 2 - Study Design

Five foods are used in studies described herein. The nutrients of the foods are analyzed and presented Table 2.

**Table 2. Average of analyzed nutrient profiles of the five foods utilized in the study**

| Nutrient | Composition H | Composition P1 | Composition P2 | Composition E1 | Composition E2 |
|---|---|---|---|---|---|
| Crude Protein, % | 29.4 | 27.9 | 30.2 | 32.2 | 29.43 |
| Fat, % | 17.6 | 14.2 | 15.1 | 21.4 | 16.42 |
| Ca, % | 1.43 | 1.46 | 1.37 | 1.48 | 1.35 |
| P, % | 1.2 | 0.96 | 1.02 | 1.12 | 1.24 |
| EPA, % | 0.31 | <0.01 | <0.01 | 0.13 | 0.13 |
| DHA% | 0.19 | 0.01 | <0.01 | 0.13 | 0.095 |
| Linoleic Acid, % | 3.8 | 2.14 | 2.58 | 3.7 | 2.85 |
| Total n-3 fatty acids, % | 1.4 | 0.19 | 0.12 | 0.49 | 0.35 |
| Total n-6 fatty acids, % | 3.7 | 2.6 | 1.73 | 3.76 | 2.84 |
| Taurine, % | 0.14 | 0.08 | 0.08 | 0.18 | 0.15 |
| Carnitine, ppm | 312.1 | No analysis | No analysis | 32.2 | No analysis |
| Methionine, % | 1.3 | 0.52 | 0.6 | 0.78 | 0.6 |
| Cystine, % | 0.4 | 0.39 | 0.43 | 0.34 | 0.29 |
| Manganese, % | 94.5 | 64 | 62 | 61.1 | 52 |
| Vitamin E, IU/kg | 816 | 43.8 | 60.5 | 300 | 311.6 |
| Vitamin C, ppm | 168.6 | <10 | No analysis | 11.6 | No analysis |
| Choline, ppm | 4876 | No analysis | 1822 | 3199 | 888 |
| Moisture, % | 6.09 | 7.44 | 6.6 | 6.6 | 7.18 |

Composition H is the composition that is the subject of this application. The other four compositions are available commercially. Compositions P1 and P2 are the same brand of food, but produced in different lots. Compositions E1 and E2 originate from the same manufacturer, but composition E1 is marketed to small breed dogs, and composition E2 is marketed for medium breed dogs.

22 dams are divided into three groups to receive either composition H, P1 or E1 for at least 10 days prior to conception. Dams are maintained in group lodging until confirmed pregnant via palpation, and are then moved to maternity lodging. Puppies from dams are kept on same foods the dams are originally assigned until the puppies are weaned. Following weaning, forty-eight puppies produced by dams fed composition P1, 16 puppies produced from dams fed composition E1, and 16 puppies produced from dams fed composition H are randomly selected for further testing.

Following weaning, of the 48 puppies from dams fed composition P1, 16 puppies are each switched to either one of compositions H, P2, or E2. Following weaning, the 16 puppies from dams fed composition E1 are switched to composition E2. The 16 puppies from dams fed composition H are provided composition H post weaning.

During a 10 month test period, blood samples are drawn routinely for analysis of health biomarkers as well as for proteomic and genomic assays. Dogs are scanned by DXA at 2, 4, 6, 9 and 12 months of age to document changes in body composition and bone density. In addition, at 2, 4, 6, 9 and 12 months of age, puppies have electroretinograms performed to assess retinal development. Brain imaging studies are also performed at 2, 6 and 12 months of age to assess neurological growth and development. Finally, cognitive and motor skill testing is performed continuously throughout the study to assess neurological (cognitive and motor skill) development. Throughout the duration of the study, body weights are recorded weekly and food intake of the group housed puppies is recorded daily.

### Example 3

### Canine Neurological Development

**Electroretinograms (ERGs)** are performed on the puppies of Example 2 at various time points with handheld, portable equipment, including a miniature Ganzfeld type of stimulator. Animals are dark adapted for at least one hour, then anesthetized using medetomidine (0.1 mg/kg IM) and ketamine (5 mg/kg IM) under red light. Pupils are maximally dilated using tropicamide 1% (Tropicamide Ophthalmic Solution USP, Alcon Lab. Inc., Fort Worth, Texas) and eyes anesthetized with proparacaine hydrochloride 0.5% (Alcaine, Alcon Lab. Inc., Fort Worth, Texas). A Jet contact lens electrode is positioned on the eye, and cushioned with methylcellulose (Methocel, Ciba Vision, Grosswallstadt, Germany). Subdermal platinum needle electrodes are placed at the occiput and approximately 0.5 cm from the lateral canthus of the eye and serve as ground and reference electrodes, respectively. The standard flash (SF) for this instrument (0 log) is 1.7 cd s/m2, produced by white LEDs. The flash intensity can be changed in 0.3 log steps, from -3.0 log to +1.2 log units. For scotopic stimulation in this study -3.0 log, 0 log and + 1.2 log with SF is used. After 10 minutes of light adaptation (using ordinary fluorescent lights in the room) photopic recordings are performed at 0 and +1.2 log, and finally, responses to 30 Hz flicker stimulation are studied. All recordings are saved on the ERG unit and later transported to a computer for further evaluation.

**Analysis of first ERG and initial positional discrimination with reversal:** At the first time point (8 weeks of age) only a single analysis is conducted for the ERG and spectroscopy measures. The rationale is that the puppies in the postnatal food groups are maintained on the dam food until reaching the test facility. Because ERG and MRI data are acquired approximately one week within reaching the test facility, the postnatal groups are combined into a single group (N=48). The assumption is that these measures would be representative of the food the puppies received prior to reaching the test facility.

At the second time point (4 months of age) the ERG is investigated using ANOVA. A significance level of 0.05 is required for statistical significance, however, the results are characterized as marginally significant if they achieve a significance level of 0.10.

Cognitive data for positional discrimination and reversal is investigated by analysis of variance (ANOVA), with food group serving as a between-subject variable. A significance level of 0.05 is required for statistical significance; however, the results are characterized as marginally significant if they achieve a significance level of 0.10.

**Statistical analysis for object discrimination and reversal:** Statistical analyses are performed using SPSS (version 10). The Object Discrimination data are examined using three analyses of variance (ANOVAs) produced with the GLM repeated measures procedure to evaluate differences between the five groups. The 30 Hz ERG data are analyzed with the GLM option for both the scotopic and photopic tests, while the remaining ERG data are analyzed in three multivariate ANOVAs (MANOVAs). In all cases, conformity with the GLM normality and homogeneity of variance is assessed using the Shapiro-Wilk test for the former (significance level set at α = .05) and the Levene and (where appropriate) Box tests (both α = .001) for the latter. Failure to fully satisfy either assumption is addressed by using whichever transformation (square root or logarithmic) most reduce the number of significant violations, and the significance level for the omnibus F test is also reduced to α = .025 if the transformation does not substantially eliminate them. As the ANOVA is generally a robust procedure, at least in regard to heterogeneity of variance, these efforts to correct for bias probably make the overall test conservative in nature, *i.e.* reduce the likelihood of false positives (Type I error). At the same time, the consequent increase in the probability of incorrectly rejecting real findings as non-significant (Type II error) is addressed by identifying as "marginally significant" those effects that would be flagged in a "typical" analysis where *p* ≤ .05, thus preserving the opportunity to explore other findings of possible interest. Significant results are explored further with pairwise comparisons conducted using either Tukey's Least Significant Difference (LSD), Dunnett's test or the Tamhane T2 tests depending on the heterogeneity of variances and adjusted for the number of comparisons (Bonferroni correction).

Results for cognitive and electroretinogram data suggest that both Compositions H, E1 and E2 may improve trainability and learning ability in puppies, as well as retinal function, whether fed to the dams prior to breeding and throughout lactation and growth, or when initiated after weaning when dams were fed Composition P1.

With regard to postweaning puppies fed one of three foods from dams that are fed exclusively Composition P1, a marginally significant food by group effect is found in the positional and reversal analysis [F(2,45)=2.9922, p=0.060245]. Post-hoc Fisher's indicate that compared to Composition P2, the groups fed Compositions H (p=0.024683) and Composition E2 (p=0.074957) groups commit fewer errors overall. The ANOVA also reveals a highly significant effect of task [F(1,45)=81.4819, p<0.001], which is a result of increased errors to acquire the reversal than the initial learning. Additionally, a task by group interaction is found [F(1,45)=5.1603, p=0.009602]. Individual one-way ANOVAs are conducted for each task to determine the nature of this interaction. No group effect is found on the positional task; however, a significant group effect is found on the reversal [F(2,45)=6.3878, p=0.003614]. Post-hoc Bonferroni's indicate that the groups fed Composition H (p=0.00318) and Composition E2 (p=0.04129) commit fewer errors on the reversal compared to groups fed Composition P2. The analysis examining the number of reversals acquired during the multiple reversal phase reveal a marginally significant group effect [F(2,45)=2.692, p=0.078628]. Post-hoc Fisher's indicate that the group fed Composition H acquires more reversals than both Composition P2 (p=0.067566) and Composition E2 (p=0.039307) groups.

**Preweaning treatment condition:** This is a cognitive comparison of puppies fed the same diet as their dams. A significant food group by task interaction is found in the positional and reversal analysis [F(2,44)=3.7114, p=0.032400] and a highly significant effect of task [F(1,44)=89.3907, p<0.001]. The task effect is a result of increased errors to acquire the reversal compared to the positional problem. Individual one-way ANOVAs are conducted for each task to determine the nature of food group by task interaction. No group effect is found on the positional task; however, a marginally significant group effect is found on the reversal [F(2,44)=3.0436, p=0.057807]. Post-hoc Fisher's indicated that the group fed Composition E2 committed fewer errors on the reversal compared to both Composition P2 (p=0.021493) and Composition H (p=0.082970). No effect is found on the analysis examining the effect of group on number of reversals acquired during the multiple-reversal phase.

In an object discrimination protocol, puppies learn to respond to one of two objects over 10 sessions. In the reversal phase the rewarded objects are switched so that the previously neutral object is rewarded in the reversal phase, and the previously rewarded object is no longer associated with reward. These analyses compare performance (number of correct responses over 100 trials) on the initial acquisition with the score on the reversal test.

Effects of Postweaning Only Diet: There is no effect on puppy cognitive development when all puppies are fed Composition P1 during the maternal period and then switched to other foods.

Effects of Preweaning/Postweaning Diet: This is a comparison made of the puppies that are fed the same food as mothers. Post hoc comparisons reveal a marginally significant (P<0.1) enhancement of performance (relative to Composition P1) in the initial acquisition phase for animals fed the Composition H and in the reversal phase for dogs fed Composition E2. Thus, in summary, the magnitude of the cognitive effect appears to vary with task given and with feeding regimen and reaches statistical significance in a few cases.

ERG results also support the conclusion that feeding of foods which comprise added DHA prior to or after weaning helps increase retinal function in growing puppies. Specifically, preliminary investigation shows that the effect of added DHA to maternal foods is important in improvement of amplitude of b-wave in the scotopic range of vision. This effect continues well into the post weaning period for the maternal effects and also appears in the puppies transitioned to foods comprising DHA following a maternal food that did not contain DHA. This would indicate that addition of DHA improved activity of the rods which could result in better visual acuity in instances of low-light.

### Example 4

### Bone Development

Bone development is tested in the puppies of Example 2. Data suggests that bone turnover may be reduced by administration of Composition H, which may lead to healthier bone development.

Serum samples are taken at four months and are frozen. Samples are thawed and subjected to assay as previously described (Yamka, et al. Intern J Appl Res Vet Med, Vol 4, No. 3, 2006) and in accordance with Metra BAP EIA, catalog number 8012 from Quidel (San Diego, CA).

One way analysis of variance of bone alkaline phosphatase (BAP) reveals a decreased activity of BAP in all groups compared to puppies fed Composition P1-P2. This reasonably suggests the possibility of decreased bone turnover and a propensity for more dense bone.

### Example 5

### Body Composition

Dual energy x-ray absorptiometry is performed on the puppies of Example 2 under anesthesia at 2, 4, 6, 9 and 12 months of age with the use of a LUNAR instrument (General Electric). Whole body composition is determined at each of these time points. Outcomes measured are bone mineral density, bone mineral content, lean mass, total fat mass, % fat mass, lean + bone content. ANOVA is performed at each time point to determine significant differences between groups. Body weight is obtained using an electronic scale each week for each puppy.

### Results:

**Weight gain birth to weaning**-Puppies born to the Composition H fed dams (330 gram average) are lighter than those born to Composition P1 (355 grams) or Composition E1 (362 grams) fed dams. This difference is not significant at birth between groups but at 4 weeks the ANOVA approaches significance at P=0.07. At weaning, there is a significant difference in weight between groups with puppies from Composition P1 (3060 grams) or Composition E1 (3080 grams) fed dams weighing significantly more than Composition H fed dams (2540 grams; P<0.05). Average daily weight gain is 47,38 and 47 grams/day for puppies from P1, H and E2 fed dams respectively, which is significantly different between groups (P<0.05).

**Weaning (58 days) to 4 months of age**-There is a significant difference in weights between groups at weaning as noted above. By 4 months of age there is a significant difference between means weight of the puppies as calculated by a one way ANOVA:

| | | |
|---|---|---|
| P1 Dams-P2 puppies | (P2-P2) | 6194 grams average |
| P1 Dams-H puppies | (P1-H) | 5919 grams average |
| P1 Dams-E2 puppies | (P1-E2) | 6106 grams average |
| E1 Dams- E2 puppies | (E1-E2) | 6180 grams average |
| H Dams-H puppies | (H-H) | 5106 grams average |

When groups are compared via two tailed t-test it is found that the Composition H-H group is significantly lower than any of the other four. None of the other four groups differs from each other.

**6 months of age-** At 6 months of age a significant difference still exists between groups via one-way ANOVA with the Composition H-H group being significantly less than all the other groups:

| | |
|---|---|
| P1 Dams - P2 puppies | 9544 grams average |
| P1 Dams - H puppies | 9153 grams average |
| P1 Dams - E2 puppies | 9156 grams average |
| E2 Dams - E1 puppies | 9025 grams average |
| H Dams - H puppies | 7580 grams average |

Average weight gain during this period is as follows:

| | |
|---|---|
| P1 Dams - P2 puppies | 54.1 grams/day average |
| P1 Dams - H puppies | 50.4 grams/ day average |
| P1 Dams - E2 puppies | 51.2 grams/day average |
| E1 Dams - E2puppies | 49.2 grams/day average |
| H Dams - H puppies | 41.7 grams/day average |

This is significantly different by one-way ANOVA (P<0.05). Comparison of groups by two-tailed t-test show that the Composition H-H group is significantly lower in growth rate than the P1-H, P1-P2, or P1-E2 groups and marginally different than the E1-E2 puppies group.

**DEXA analysis-** Initial DEXA analyses are performed on the three groups of H, P1 and E1 at weaning, prior to allocation to final food groups, and are compared by one-way ANOVA. Subsequent DEXA is performed at approximately 4, 6 and 9 months of age on all groups (P1-P2, P1-H, P1-E2, H-H, and E1-E2), and are compared via one-way ANOVA:

### Total Fat

Initial at weaning: Puppies from dams from E1 had more total fat than puppies from dams fed P1, which had more total fat than puppies from dams fed H (significant difference between groups (P<0.05)).

4 months of age :
▪ E1-E2 > P1-E2 > P1-P1 > P1-H > H-H
▪ Significant difference between groups (P<0.05) 9 months of age:
▪ E1-E2 > P1-P1 > P1-E2 > P1-H > H-H
▪ Puppies fed Composition H and from dams fed Composition H are less fat at 9 months of age. Significant difference between groups (P<0.05)

### Percent Fat

Initial at weaning:
▪ E1 > P1 > H
▪ Puppies from Composition H fed dams have less percent body fat at weaning. Significant difference between groups (P<0.05)

4 months of age:
▪ E1-E2 > P1-E2 >P1-P1 > P1-H > H-H
▪ Significant difference between groups (P<0.05)

9 months of age:
▪ E1-E2 > P1-E2 >P1-P1 > P1-H > H-H
▪ Puppies fed Composition H and from Composition H fed dams have less percent body fat at 9 months of age. Significant difference between groups (P<0.05)

## Claims

1. A pet food composition for a therapeutic use in promoting bone development in a canine
the composition comprising:
0.8 to 1.6% methionine;
50 to 200 ppm manganese;
0.1 to 0.5% DHA;
0.1 to 0.7% EPA; and
2500 to 7500 ppm choline; and
wherein the canine is born of a dam fed the composition prior to and during pregnancy; and the canine is fed the composition prior to weaning.

2. The composition of claim 1 for the use of claim 1 comprising 1000 to 2000 ppm taurine.

3. The composition of any one of the preceding claims for the use of any one of the preceding claims comprising 2.5 to 6 % linoleic acid.

4. The composition of any one of the preceding claims for the use of any one of the preceding claims comprising 1 to 3% total n-3 fatty acids.

5. The composition of any one of the preceding claims for the use of any one of the preceding claims comprising 50 to 1200 IU/kg vitamin E.

6. The composition of any one of the preceding claims for the use of any one of the preceding claims comprising 50 to 500 ppm vitamin C.

7. The composition of any one of the preceding claims for the use of any one of the preceding claims comprising 2.2 to 7 g lysine/1000 kcal.

8. The composition of any one of the preceding claims for the use of any one of the preceding claims comprising 0 to 90% by weight of carbohydrates.

9. The composition of any one of the preceding claims for the use of any one of the preceding claims comprising 5% to 70% by weight protein.

10. The composition of any one of the preceding claims for the use of any one of the preceding claims comprising 2% to 50% by weight of fat.

11. The composition of any one of the preceding claims for the use of any one of the preceding claims comprising 0.1% to 20% by weight of total dietary fiber.

12. The composition of any one of the preceding claims for the use of any one of the preceding claims comprising 2.5 to 7 g lysine/1000 kcal.

13. The composition of any one of the preceding claims for the use of any one of the preceding claims comprising 100 ppm to 500 ppm carnitine.

14. The composition of any one of the preceding claims for the use of any one of the preceding claims wherein the canine is a puppy.

15. The composition claim 14 for the use of claim 14 wherein the puppy is further fed the composition post weaning.

## Patentansprüche

1. Heimtierfutter-Zusammensetzung zur therapeutischen Verwendung bei der Förderung der Knochenbildung bei einem Hund, wobei die Zusammensetzung Folgendes umfasst:
0,8 bis 1,6 % Methionin;
50 bis 200 ppm Mangan;
0,1 bis 0,5 % DHA;
0,1 bis 0,7 % EPA und
2500 bis 7500 ppm Cholin; und
wobei der Hund von einem Muttertier geboren wird, dem die Zusammensetzung vor und während der Trächtigkeit gefüttert wird; und dem Hund die Zusammensetzung vor dem Absetzen gefüttert wird.

2. Zusammensetzung nach Anspruch 1 zur Verwendung nach Anspruch 1, umfassend 1000 bis 2000 ppm Taurin.

3. Zusammensetzung nach einem der vorangehenden Ansprüche zur Verwendung nach einem der vorangehenden Ansprüche, umfassend 2,5 bis 6 % Linolsäure.

4. Zusammensetzung nach einem der vorangehenden Ansprüche zur Verwendung nach einem der vorangehenden Ansprüche, umfassend 1 bis 3 % Gesamt-Omega-3-Fettsäuren.

5. Zusammensetzung nach einem der vorangehenden Ansprüche zur Verwendung nach einem der vorangehenden Ansprüche, umfassend 50 bis 1200 IE/kg Vitamin E.

6. Zusammensetzung nach einem der vorangehenden Ansprüche zur Verwendung nach einem der vorangehenden Ansprüche, umfassend 50 bis 500 ppm Vitamin C.

7. Zusammensetzung nach einem der vorangehenden Ansprüche zur Verwendung nach einem der vorangehenden Ansprüche, umfassend 2,2 bis 7 g Lysin/1000 kcal.

8. Zusammensetzung nach einem der vorangehenden Ansprüche zur Verwendung nach einem der vorangehenden Ansprüche, umfassend 0 bis 90 Gew.-% Kohlenhydrate.

9. Zusammensetzung nach einem der vorangehenden Ansprüche zur Verwendung nach einem der vorangehenden Ansprüche, umfassend 5 bis 70 Gew.-% Protein.

10. Zusammensetzung nach einem der vorangehenden Ansprüche zur Verwendung nach einem der vorangehenden Ansprüche, umfassend 2 bis 50 Gew.-% Fett.

11. Zusammensetzung nach einem der vorangehenden Ansprüche zur Verwendung nach einem der vorangehenden Ansprüche, umfassend 0,1 bis 20 Gew.-% Gesamtballaststoffe.

12. Zusammensetzung nach einem der vorangehenden Ansprüche zur Verwendung nach einem der vorangehenden Ansprüche, umfassend 2,5 bis 7 g Lysin/1000 kcal.

13. Zusammensetzung nach einem der vorangehenden Ansprüche zur Verwendung nach einem der vorangehenden Ansprüche, umfassend 100 bis 500 ppm Carnitin.

14. Zusammensetzung nach einem der vorangehenden Ansprüche zur Verwendung nach einem der vorangehenden Ansprüche, wobei der Hund ein Welpe ist.

15. Zusammensetzung nach Anspruch 14 zur Verwendung nach Anspruch 14, wobei dem Welpen die Zusammensetzung ferner nach dem Absetzen gefüttert wird.

## Revendications

1. Composition alimentaire pour animaux familiers utilisée à des fins thérapeutiques pour favoriser le développement osseux chez un canin, la composition comprenant :
de 0,8 à 1,6 % de méthionine ;
de 50 à 200 ppm de manganèse ;
de 0,1 à 0,5 % de DHA ;
de 0,1 à 0,7 % d'EPA ; et
de 2 500 à 7 500 de choline ; et
le canin étant né d'une mère nourrie avec la composition avant et pendant la gestation et
le canin étant nourri avec la composition après le sevrage.

2. Composition de la revendication 1 pour l'utilisation de la revendication 1, qui comprend de 1 000 à 2 000 ppm de taurine.

3. Composition de l'une quelconque des revendications précédentes pour l'utilisation de l'une quelconque des revendications précédentes, qui comprend de 2,5 à 6 % d'acide linoléique.

4. Composition de l'une quelconque des revendications précédentes pour l'utilisation de l'une quelconque des revendications précédentes, qui comprend de 1 à 3 % d'acides gras n-3 totaux.

5. Composition de l'une quelconque des revendications précédentes pour l'utilisation de l'une quelconque des revendications précédentes, qui comprend de 50 à 1 200 Ul/kg de vitamine E.

6. Composition de l'une quelconque des revendications précédentes pour l'utilisation de l'une quelconque des revendications précédentes, qui comprend de 50 à 500 ppm de vitamine C.

7. Composition de l'une quelconque des revendications précédentes pour l'utilisation de l'une quelconque des revendications précédentes, qui comprend de 2,2 à 7 g de lysine/1 000 kcal.

8. Composition de l'une quelconque des revendications précédentes pour l'utilisation de l'une quelconque des revendications précédentes, qui comprend de 0 à 90 % en poids d'hydrates de carbone.

9. Composition de l'une quelconque des revendications précédentes pour l'utilisation de l'une quelconque des revendications précédentes, qui comprend de 5 à 70 % en poids de protéines.

10. Composition de l'une quelconque des revendications précédentes pour l'utilisation de l'une quelconque des revendications précédentes, qui comprend de 2 à 50 % en poids de matières grasses.

11. Composition de l'une quelconque des revendications précédentes pour l'utilisation de l'une quelconque des revendications précédentes, qui comprend de 0,1 à 20 % de fibres alimentaires totales.

12. Composition de l'une quelconque des revendications précédentes pour l'utilisation de l'une quelconque des revendications précédentes, qui comprend de 2,5 à 7 g de lysine/1 000 kcal.

13. Composition de l'une quelconque des revendications précédentes pour l'utilisation de l'une quelconque des revendications précédentes, qui comprend de 100 à 500 ppm de carnitine.

14. Composition de l'une quelconque des revendications précédentes pour l'utilisation de l'une quelconque des revendications précédentes, le canin étant un chiot.

15. Composition de la revendication 14 pour l'utilisation de la revendication 14, le chiot étant en outre nourri avec la composition après le sevrage.
